# EUROPEAN PATENT APPLICATION

(11) **EP 3 069 725 A1**
(43) Date of publication of application: **21.09.2016**
(21) Application number: 15159405.8
(22) Date of filing: 17.03.2015
(51) Int. Cl.: A61K 36/896, A61P 35/00

(54) **GLORIOSA SUPERBA L. EXTRACTS, COMPOSITIONS AND USE THEREOF**

(71) Applicant: INDENA S.p.A., 20139 Milano (IT); Universiteit Antwerpen, 2000 Antwerpen (BE)
(72) Inventor: PIETERS, Luc, 2000 Antwerp (BE); APERS, Sandra, 2870 Puurs (BE); CAPISTRANO, Rica, 2550 Kontich (BE)
(74) Representative: Bianchetti Bracco Minoja S.r.l.

(57) **Abstract**

The present invention relates to *Gloriosa superba* colchicine-poor extracts, useful in the treatment of cancers, in particular of pancreatic cancer.

## Description

### Technical field of the invention

The present invention relates to *Gloriosa superba* L. colchicine-poor extracts, composition comprising the extracts and the use of the extracts in the treatment of cancers, in particular of pancreatic cancer.

### Background of the invention

*Gloriosa superba* L., commonly called glory lily, flame lily, climbing lily or creeping lily, belongs to the family of the Liliaceae. *G. superba* is native to tropical Africa, south-eastern Asia and India and is now widely cultivated throughout the world as an ornamental plant. It is a climbing herb with tendril to cling onto other plants that can grow up to 6 m long. The leaves are dark green and glossy and they occur in whorls of 3 to 4, alternate, sessile, lanceolate and spear shaped with curved end. Flowers are large, solitary at the ends of branches, bicoloured yellow and red, and have six erect petals. The fruits are ellipsoid capsules that split open to release several smooth red seeds. The glory lily is a tuberous plant with V or L-shaped, finger-like tubers that are pure white when young, becoming brown with the age.

*G. superba* is a well-known product that has been in regular demand amongst practitioners of traditional medicine in tropical Africa and Asia. It is one of the most important exported medicinal plants of India and is still often used in Ayurveda. Five different plant parts of *G*. *superba* are cited as important in ethnobotanical applications, i.e. leaves, seeds, unripe fruits, tubers and the whole plant. From all plant parts the tuber is the most frequently used. It is used as a germicide, to cure ulcers, piles, haemorrhoids, inflammation, scrofula, leprosy, dyspepsia, worm infections, flatulence, intermittent fevers, debilitating arthritis and snake bites. The tubers are also boiled with sesame oil and applied twice a day on the joints to reduce arthritis pain. The seeds are used for relieving rheumatic pain and as a muscle relaxant.

*G. superba* is one of the seven semi-poisonous drugs in the Indian traditional medicine, which cure many ailments but may prove fatal on misuse. Traditional healers seem to be aware of its toxicity as the amounts they prescribe to their patients are such that the toxic symptoms are minimised. The toxins of *G*. *superba* have an inhibitory action on cellular division resulting in diarrhoea, depressant action on bone marrow and alopecia. Its poisonous properties are mainly due to colchicine.

The best known constituent in *G*. *superba* is the alkaloid colchicine. Due to the anti-inflammatory activity it is now still used for the treatment of gout and the autoimmune disease Familial Mediterranean Fever (FMF).

Colchicine blocks cyclooxygenase-2 (COX-2) activity, prostaglandin E₂ and thromboxane A₂ synthesis of mononuclear phagocytes with subsequent reduction of swelling and pain. Therefore, it is also an effective drug treatment for intense pain associated with a gout attack and FMF.

Colchicine binds to tubulin and blocks microtubules formation. The mechanism of action at the level of the microtubules makes colchicine very interesting for the treatment of cancer. Cancer cells are more susceptible than normal cells considering their high division rate. Drugs that interact with tubulin polymerisation are divided into two groups: the stabilising and the destabilising agents. Colchicine belongs to the group of the destabilising agents, because of its mode of action in higher concentrations, which is inducing depolymerisation of microtubules. For pure colchicine to have an anticancer effect the dose to be given to humans was reported to be so high it was excluded for toxicity reasons.

Different colchicine derivatives have been isolated and identified from G. *superba.* Scheme 1 shows the structures of the reported alkaloids.

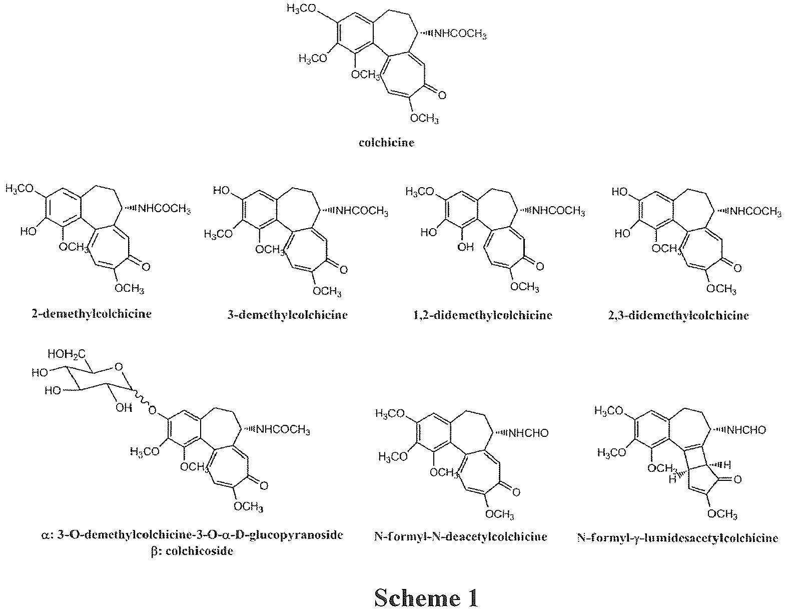

Studies on the cytotoxic activity of G. *superba* extracts and pure alkaloids have also been conducted. The biological effects on cancer cells of modified colchicines were studied in 1981. Various colchicine derivatives have been synthesised, after which the activity *in vitro* and *in vivo* was compared with colchicine. This revealed a correlation between the *in vivo* potency and toxicity. The 3-*O*-demethylcolchicine (3.6 µmol/kg) structure still had a reasonable potency compared to colchicine (0.4 µmol/kg), in contrast to the 2-*O*-demethylcolchicine (13.8 µmol/kg). Colchicoside was not *active in vitro* but was not tested *in vivo.* Colchicoside, however, can act as a prodrug of the glycosidic 3-*O*-demethylcolchicine, which may explain the inactivity *in vitro.*

In 2000 an article was published on the cytotoxicity of some medicinal plants from Tanzanian traditional medicine. The methanol extracts from the plants were tested at a concentration of 10 and 100 µg/mL in three cancer cell lines (HeLa, HT-29 and A431). The extract of G. *superba* reduced the cell proliferation in each case by at least 50% compared to the control cells, most likely due to the presence of colchicine.

In Europe, pancreatic cancer is the tenth most frequent cancer and the eighth leading cause of cancer-related death. Pancreatic cancer has an extremely poor prognosis and is one of the deadliest types of cancer. Most pancreatic cancer patients will die within the first year of diagnosis and only 6% will survive five years. The lack of progress in primary prevention, early diagnosis and treatment underscores the need for additional efforts in pancreatic cancer research.

The pancreas contains two types of glands: the exocrine glands that produce enzymes that help to digest food, and the endocrine glands that produce important hormones such as insulin. They develop completely different types of tumours with distinct risk factors, symptoms, diagnostics tests, treatment and survival rate. The exocrine tumours are by far the most common type of pancreatic cancer. The cause of pancreatic cancer is not well-known, but there are several factors that increase the risk, such as obesity, tobacco use, family history, certain inherited syndromes, etc. The prognosis is largely determined by the stage of the disease at diagnosis, whether there is lymph node involvement, and the extent of spread, i.e. local as well as to distant organs. The median survival ranges from 4.5 months for the most advanced stage to 24.1 months for the earliest stage.

The treatment is largely determined by whether the tumour can be removed surgically. This is the only treatment that offers a chance of cure for the patients. Postoperative (adjuvant) chemotherapy either alone or in combination with radiation has been proven to improve progression-free and overall survival in both randomised controlled trials and observational studies. Gemcitabine is usually the recommended first-line chemotherapy for pancreatic cancer patients. It is given alone or in combination with other drugs or radiotherapy.

Because of the extreme poor prognosis of pancreatic cancer, and because conventional cancer therapy only has little impact on this disease, there is still the need to find new therapies useful in the treatment of cancer, in particular of pancreatic cancer.

### Summary of the invention

The present invention relates to a *Gloriosa superba* L. colchicine-poor extract.

The invention also concerns a composition comprising the *Gloriosa superba* L. colchicine-poor extract and at least a physiological acceptable carrier and/or excipient.

Furthermore, the invention regards the use of a *Gloriosa superba* L. colchicine-poor extract in the treatment of cancers, in particular of pancreatic cancer.

### Detailed description of the invention

The present invention relates to a *Gloriosa superba* L. colchicine-poor extract.

The *Gloriosa superba* L. colchicine-poor extract has a relatively low content of colchicine and it comprises colchicine derivatives, especially colchicoside.

The total colchicine derivatives content preferably is at least 1% w/w, more preferably at least 2% w/w.

The colchicine content of the extract is up to 1% w/w, preferably it is up to 0.5% w/w, more preferably up to 0.1% w/w.

The colchicine derivatives comprised in the *Gloriosa superba* L. colchicine-poor extract may be colchicoside, 3-*O*-demethylcolchicine; especially colchicoside.

The colchicoside content of the extract preferably is at least 1% w/w, more preferably at least 2% w/w.

The 3-*O*-demethylcolchicine content of the extract is preferably below 2% w/w, more preferably below 1% w/w.

In a preferred embodiment, the *Gloriosa superba* L. colchicine-poor extract comprises up to 0.1% w/w of colchicine, at least 2% w/w of colchicoside and up to 0.5% w/w of 3-*O*-demethylcolchicine.

It has been surprisingly found that a *Gloriosa superba* L. colchicine-poor extract is useful in the treatment of cancer, in particular pancreatic cancer.

The colchicine-poor extract is preferably prepared from an ethanolic extract of *Gloriosa superba* L., preferably from an at least 50% ethanol extract, more preferably from an 80% ethanol extract.

In a preferred embodiment, the extract may be prepared from seeds.

The *Gloriosa superba* L. colchicine-poor extract is prepared form a 80% ethanol extract of *Gloriosa superba* L. obtained from seeds.

A process comprising the following steps may be used for preparing the *Gloriosa superba* L. colchicine-poor extract according to the invention:
a) dissolving a *Gloriosa superba* L. crude dry ethanolic extract in 5% acetic acid;
b) extracting the solution obtained in step a) three times with diethyl ether;
c) extracting the aqueous fraction (GS1B) obtained in step b) three times with methylene chloride to obtain the aqueous fraction GS2B.

The present invention relates to a composition comprising a *Gloriosa superba* L. colchicine-poor extract and at least a physiologically acceptable carrier and/or excipient.

The compositions according to the invention may be prepared according to conventional techniques as described for example in Remington's Pharmaceutical Handbook, Mack Publishing Co., NY USA. In particular, the compositions of the invention may be prepared according to conventional techniques known for formulations comprising herbal ingredients.

Furthermore, the invention relates to the use of a colchicine-poor extract of *Gloriosa superba* L. for the treatment of cancer, particularly of pancreatic cancer.

The colchicine-poor *Gloriosa superba* L. extract may be used for the treatment of cancer, particularly of pancreatic cancer, also in combination with one or more chemotherapy drug used to treat cancer, such as gemcitabine.

The following non-limitative examples further describe the invention.

### Examples

### Example 1 - Preparation of a Gloriosa superba L. crude extract (GS)

The seeds of *Gloriosa superba* L. were dried an extra two weeks in an oven at a temperature of 45 °C. The seeds were then ground and sieved through a 2 mm sieve. The ground seeds (5.3 kg) were extracted exhaustively and consecutively by percolation and maceration with 95 L of 80% ethanol at room temperature. The ethanol was removed under reduced pressure at 40 °C and the aqueous extract was lyophilised. The yield of the crude extract was 846.7 g. The extract contained colchicine 3.22% w/w, colchicoside 2.52% w/w and 3-*O*-demetylcolchicine 1.32% w/w.

### Example 2 - Preparation of a Gloriosa superba L. colchicine-poor extract (GS2B)

Liquid-liquid partition was performed according to the scheme reported in Scheme 2.

About 50 g of the crude dry extract (prepared according to example 1) was dissolved in 300 mL 5% acetic acid (pH 2.37).

This solution was extracted three times with 300 mL diethyl ether.

The organic fraction GS1A was concentrated under reduced pressure at 40 °C to 150 mL and washed once with 150 mL freshly prepared 5% acetic acid.

The aqueous fractions (GS1B) were pooled and concentrated under reduced pressure at 40 °C to 300 mL. Fraction GS1B was then extracted three times with 300 mL methylene chloride.

After concentrating the methylene chloride fraction to 300 mL, this organic fraction (GS2A) was washed with 300 mL freshly prepared 5% acetic acid.

The aqueous fraction (GS2B) was obtained containing colchicine 0.07% w/w, colchicoside 2.26% w/w and 3-*O*-demethylcolchicine 0.46% w/w.

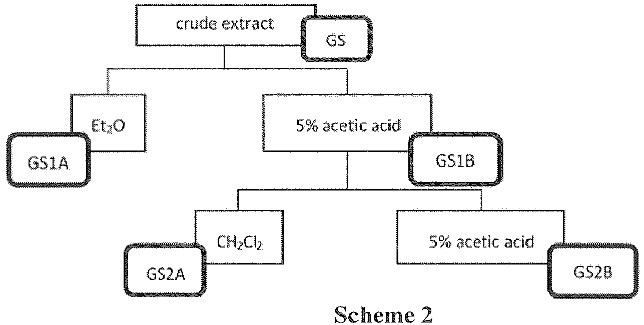

### Example 3 - In vitro cytotoxicity

Using the SRB assay GS prepared according to Example 1 and GS2B prepared according to Example 2 were tested on murine pancreatic cancer cells PANC02.

The sulforhodamine B (SRB) assay was performed at the Centre for Oncological Research (CORE) of the University of Antwerp. The SRB assay is a colorimetric assay used for cell density determination, based on the measurements of cellular protein content. It is used to measure drug-induced cytotoxicity. The SRB assay was performed according to the method of Papazisis et al, with minor modification. Cells were harvested from exponential phase cultures by trypsinisation and counted with the Scepter™ 2.0 (Merck Millipore, Darmstadt, Germany), which is an automated cell counter based on the Coulter principle. Optimal seeding densities were determined to ensure exponential growth and were incubation time and cell line dependent. Table 1 shows the used cell density per cell line and incubation time. In each experiment 7 different concentrations of plant extract were tested on the different cell line and 6 replicate wells per concentration were used. Two different incubation times were evaluated, i.e. 24 h and 72 h. The stock solution was prepared by dissolving the plant extracts in sterile water and by passing the solution through a 0.22 µm filter. After incubation, culture media was removed prior to fixation, which was done by adding 200 µL of 10% cold trichloroacetic acid onto the cells. After one hour at 4 °C, the cells were washed five times with deionised water. The cells were then stained with 200 µL 0.1% SRB in 1% acetic acid for 15 min at room temperature. To remove the unbound stain the cells were washed with 1% acetic acid four times. The plates were left to dry at room temperature. The protein-bound stain was re-dissolved with 200 µL 10m*M* unbuffered tris and transferred to 96-well plates for the optical density (OD) reading at 540 nm (Biorad 550 microplate reader and iMark Microplate Absorbance Reader, Nazareth, Belgium).

**Table 1**

| **Cell lines** | **Cell density** (**cells**/**well**) | |
|---|---|---|
| | **24 h** | **72 h** |
| **PANC02** | 500 | 500 |

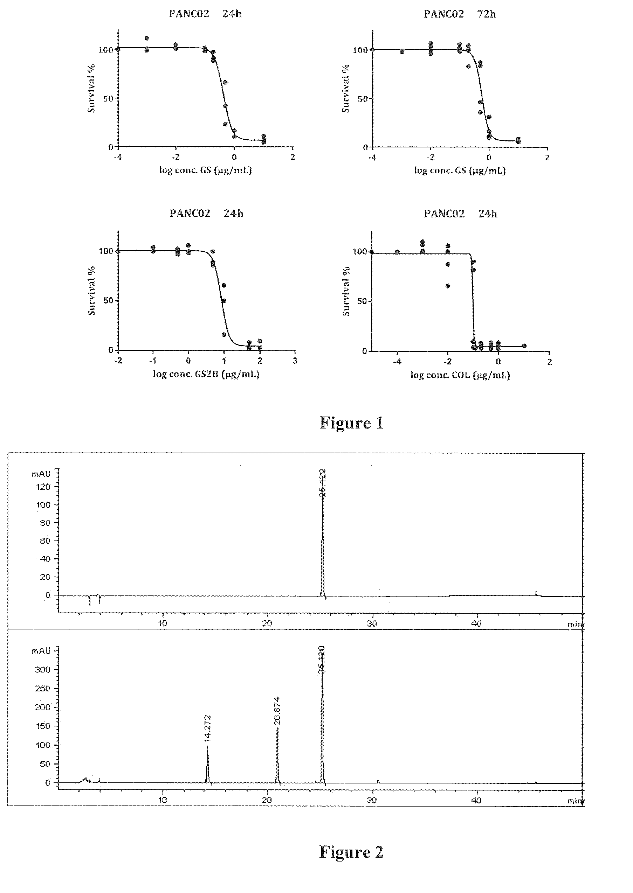

For the PANC02 cell line, the used concentrations of GS ranged from 0.001-10 µg/mL. At least three individual experiments were performed. The colchicine-poor extract (GS2B) and colchicine (COL) were tested *in vitro* on the PANC02 cells, but using only one incubation time. Also at least three experiments were performed. The concentration ranged from 0.1 - 100 µg/mL for GS2B and from 0.0001 - 10 µg/mL for COL. The results of all these experiments are displayed in Figure 1. The calculated IC₅₀ values for GS was 0.45 ± 0.03 µg/mL with an incubation time of 24 h. IC₅₀ values of 9.49 ± 0.41 and 0.098 ± 0.004 µg/mL were determined for GS2B and COL, respectively, with an incubation time of 24 h.

The extract GS2B has a relatively low level of colchicine and a relatively high content of colchicine derivatives, especially colchicoside. This extract was tested *in vitro,* but since colchicoside needs to be activated *in vivo,* not surprisingly the IC₅₀ was much higher for this extract than for GS. An IC₅₀ value of 9.49 µg/mL was observed on the PANC02 cell line, which is hundred times larger than the value observed for colchicine itself, i.e. 0.098 µg/mL. The *in vitro* cytotoxicity of GS2B is mainly due to residual colchicine and 3-*O*-demethylcolchicine.

### Example 4 - In vivo - efficacy study

The G. *superba* L. crude extract (GS) and the colchicine-poor extract (GS2B) were tested *in vivo* in a murine model.

### Materials and methods

### Cell culture

The same PANC02 cell culture was used.

### Animals

Six-to-eight weeks old female C57BL/6 mice (16-20 g body weight) were purchased from Harlan laboratories. The mice were housed in individually ventilated cages, under a 10/14 h dark/ light cycle at constant temperature and humidity and had access to tap water and food ad libitum. All animals were treated in accordance to the guidelines and regulations for use and care of animals. All mice experiments were approved by the local Ethical Committee of the University of Antwerp, approved study number: 2013-03.

### Subcutaneous tumour model

Cultures of PANC02 cells were harvested using a 0.05% trypsin solution, washed twice in sterile PBS and resuspended in sterile PBS at a concentration of 30 x 10⁶ cells per mL. The viable cells were counted by using the Muse^{®} Cell Analyzer. The mice (n = 56) were inoculated with 100 µL of the cell suspension in the right hind limb. Each mouse was randomly assigned to one of 8 groups (n = 6-8 mice per group) receiving a different treatment (Table 2). Tumour growth of the subcutaneous model was evaluated with calliper measurements from the moment the tumours became palpable and this three times a week before treatment and daily during treatment. The tumour volume was calculated by means of formula V = 0.5 x ab², with a and b being the long and short axes of the tumour, respectively.

**Table 2 - Group treatment assignment**

| n° | **Group** | # **mice** | **Treatment and dose** |
|---|---|---|---|
| 1 | Negative control | 7 | 200 µl water p.o. |
| 2 | Positive control | 7 | 60 mg/kg BW gemcitabine IP |
| 3 | *Gloriosa superba* extract (GS) | 6 | 0.3 mg/kg BW TC* |
| | | | 5.2 mg/kg BW extract p.o. |
| 4 | | 6 | 1 mg/kg BW TC* |
| | | | 17.2 mg/kg BW extract p.o. |
| 5 | | 8 | 3 mg/kg BW TC* |
| | | | 51.7 mg/kg BW extract p.o. |
| 6 | Colchicine-poor extract (GS2B) | 6 | 0.3 mg/kg BW TC* |
| | | | 18.8 mg/kg BW extract p.o. |
| 7 | | 6 | 1 mg/kg BW TC* |
| | | | 62.5 mg/kg BW extract p.o. |
| 8 | | 7 | 3 mg/kg BW TC* |
| | | | 187.5 mg/kg BW extract p.o. |

| | | | |
|---|---|---|---|
| ** Total amount of colchicine and its derivatives expressed as colchicine* | | | |

### Treatments

Treatment was administered during 10 days, starting 19 days after tumour inoculation when tumours had reached a volume of approximately 300-400 mm³. The first day of treatment was assigned "day 1". The negative control group received 200 µL water per oral gavage (p.o.) daily. The positive control group was given 3 times/week (days 1, 3, 5, 8, 10) gemcitabine (Actavis, 38 mg/mL). It was administered at a dose of 60 mg/kg BW intraperitoneally (IP). GS and GS2B were administered by oral gavage daily at a dose of 0.3, 1 and 3 mg/kg BW total amount of colchicine and its derivatives expressed as colchicine. For both extracts the total amount of colchicine and its derivatives (calculated as colchicine) was the same, but the amount of colchicine itself in the extracts was different. The amount of colchicine in GS2B was less than 0.1% (m/m). With the use of the validated analytical method described below, the percentages of colchicine and its derivatives in GS as in GS2B were analysed and amounted to 5.79% (m/m) and 1.61% (m/m) TC, respectively. Taking this percentage into account, groups 3, 4 and 5 were given 5.2, 17.2 and 51.7 mg/kg BW GS and groups 6, 7 and 8 were given 18.8, 62.5 and 187.5 mg/kg BW GS2B (Table 2). To evaluate the effect of the treatment, tumour volume was measured daily during treatment and the mice were sacrificed on day 11.

### Final analytical method

### Reference solution

About 25 mg of colchicine (97% purity) was accurately weighed into a measuring flask of 50.0 mL. This was then dissolved in 50% MeOH and ultrasonicated during 15 min. After cooling down, this solution was then diluted 12.5, 50 and 250 times resulting in concentrations of 0.002, 0.01 and 0.04 mg/mL, respectively.

### Test solution

About 100 mg of *G*. *superba* extract was accurately weighed into a measuring flask of 20.0 mL. It was dissolved in MeOH and ultrasonicated during 15 min. After cooling down, 5.0 mL of this solution was transferred into a measuring flask of 25.0 mL. Five mL of water was added into the same flask and filled up with 50% MeOH, after cooling down.

### HPLC conditions

The reference and test solution were analyzed by means of HPLC by injecting 20 µL. The following gradient was used with mobile phase A being water and B acetonitrile: 0 min, 10% B; 5 min, 10% B; 25 min, 40% B; 35 min, 100% B; 40 min, 100% B. The column used was a Phenomenex Luna 5 µm C18 100 Å (250 x 4.6 mm) with a flow rate of 1.0 mL/min. The peaks were detected at 245 nm. The resulting chromatograms of the reference material, colchicine, and the crude extract of G. *superba* are shown in Figure 2.

### Statistics

Results were expressed as mean values of parameters ± standard error (SE). The parametric ANOVA test was used to determine statistical significance, followed by a post hoc Tukey analysis to establish the statistical difference between the treatment groups.

### Results

All mice developed subcutaneous tumours in the right hind limb (100% tumour growth). Eighteen days after inoculation, the tumours had reached a mean volume of 300-400 mm³ and treatment started. On day 0, which was the day before treatment, the starting mean tumour volumes per group were plotted and tumour volumes were normalised. The mean relative tumour volumes (RTVs) were then calculated for each group for each day and plotted. The mean RTVs on day 7 and 10 were graphed and the difference between the groups was statistically determined. The percentage of tumour growth inhibition (%TGI) on day 7 and 10 were calculated according to the formula: %TGI = 1 - (RTVx - 1/RTVc - 1), with RTVx being the relative tumour volume from the treated group and RTV_{C} the relative tumour volume from the control group. The %TGI are summarized in Table 3. All animals were weighed daily during treatment and the mean body weight per group was graphed.

**Table 3**

| **Treatment** | **% TGI (7 days)** | **% TGI (10 days)** |
|---|---|---|
| **Gemcitabine** | 90% | 99% |
| **GS (0.3 mg/kg)** | 78% | 58% |
| **GS (1 mg/kg)** | 86% | 46% |
| **GS (3mg/kg)** | 101% | 74% |
| **GS2B (0.3 mg/kg)** | 74% | 60% |
| **GS2B (1 mg/kg)** | 81% | 30% |
| **GS2B (3mg/kg)** | 93% | 63% |

### Discussion

The mice were randomly divided in groups before tumour inoculation. No statistical difference (p = 0.31) between the groups was observed on day 0. The mean RTVs during the entire treatment period showed a difference between the treatment groups and the control group. A dose-effect relationship was observed for GS and GS2B, and although the response of the two lowest concentrations were quite the same, the biggest effect during the entire experiment was observed at the highest dose of both extracts. The RTV of the control group was higher than the treated groups and until day 7 the effect of gemcitabine was comparable to the effect of the extracts.

On day 7, the RTVs of all treatment groups were significantly smaller compared to the control group. On day 10, significantly smaller RTVs were still observed for the lowest and the highest dose of both extracts compared to the control group. The tumour growth inhibition was also calculated for the different groups. On day 7, all treatment groups showed %TGI above 50%, which is considered a relevant inhibition. The greatest tumour growth inhibition was observed for the highest dose of both extracts, which is comparable with gemcitabine. On day 10, a relevant tumor growth inhibition was still observed for the lowest (0.3 mg/kg) and highest (0.3 mg/kg) dose of GS with a %TGI of 58 and 74%, respectively. For GS2B, also the lowest dose and highest dose showed a relevant tumour growth inhibition with a %TGI of 60% and 63%, respectively.

The treatments did not influence the body weight. No significant weight loss was observed throughout the experiment. Although the GS (1 mg/kg) group showed a downfall on day 1, the rapid gain of weight was an indication that this weight loss was due to an accidental extra fasting day rather than an effect of the treatment.

## Claims

1. *Gloriosa superba* L. colchicine-poor extract comprising colchicine and colchicine derivatives, wherein the colchicine content is up to 1% w/w.

2. *Gloriosa superba* L. colchicine-poor extract according to claim 1, wherein the colchicine content is up to 0.5% w/w.

3. *Gloriosa superba* L. colchicine-poor extract according to claim 1 or 2, wherein the colchicine content is up to 0.1% w/w.

4. *Gloriosa superba* L. colchicine-poor extract according to claim 1, wherein the colchicine derivatives content is at least 1% w/w.

5. *Gloriosa superba* L. colchicine-poor extract according to claim 1-4, wherein the colchicine derivatives are colchicoside, 3-*O*-demethylcolchicine.

6. *Gloriosa superba* L. colchicine-poor extract according to claim 5, wherein the colchicine derivative is colchicoside.

7. *Gloriosa superba* L. colchicine-poor extract according to claim 6, wherein the colchicoside content is at least 1% w/w.

8. *Gloriosa superba* L. colchicine-poor extract according to claim 6, wherein the colchicoside content is at least 2% w/w.

9. *Gloriosa superba* L. colchicine-poor extract according to claim 5, wherein the 3-*O*-demethylcolchicine content is below 2% w/w.

10. *Gloriosa superba* L. colchicine-poor extract according to claim 5, wherein the 3-*O*-demethylcolchicine content is below 1% w/w.

11. *Gloriosa superba* L. colchicine-poor extract according to claims 1-10, wherein the colchicine content is up to 0.1% w/w, the 3-*O*-demethylcolchicine content is up to 0.5% w/w and the colchicoside content is at least 2% w/w.

12. A composition comprising a *Gloriosa superba* L. colchicine-poor extract according to claims 1-11 and at least a physiologically acceptable carrier and/or excipient.

13. *Gloriosa superba* L. colchicine-poor extract according to claims 1-11 for use in treatment of cancer.

14. *Gloriosa superba* L. colchicine-poor extract for use according to claim 13, wherein the cancer is pancreatic cancer.

15. *Gloriosa superba* L. colchicine-poor extract for use according to claim 13 or 14, in combination with one or more chemotherapy drug.

16. *Gloriosa superba* L. colchicine-poor extract for use according to claim 15, wherein the chemotherapy drug is gemcitabine.

17. A process for preparing a *Gloriosa superba* L. colchicine-poor extract according to claims 1-11 comprising the following steps:
a) dissolving a *Gloriosa superba* L. crude dry extract in 5% acetic acid;
b) extracting the solution obtained in step a) three times with diethyl ether;
c) extracting the aqueous fraction (GS1B) obtained in step b) three times with methylene chloride to obtain the aqueous fraction GS2B.
